# EUROPEAN PATENT APPLICATION

(11) **EP 3 275 863 A1**
(43) Date of publication of application: **31.01.2018**
(21) Application number: 16382373.5
(22) Date of filing: 29.07.2016
(51) Int. Cl.: C07D 213/75, C07C 275/30

(54) **COMPOUNDS FOR TREATING CONGENITAL DISORDERS OF GLYCOSYLATION**

(71) Applicant: Universidad Autónoma de Madrid, 28049 Madrid (ES); University of Bergen, 5006 Bergen (NO)
(72) Inventor: PÉREZ GONZÁLEZ, Maria Belén, E-28049 Madrid (ES); GÁMEZ ABASCAL, Maria Alejandra, E-28049 Madrid (ES); YUSTE CHECA, Patricia, 28049 Madrid (ES); BRASIL, Sandra, 28049 Madrid (ES); UGARTE PÉREZ, Magdalena, 28049 Madrid (ES); MARTÍNEZ RUIZ, Aurora, 5006 Bergen (NO); UNDERHAUG, Jarl, 5006 Bergen (NO)
(74) Representative: ABG Patentes, S.L.

(57) **Abstract**

The present invention relates to the medicinal use of small organic compounds, in particular amide and urea derivatives, for the treatment of congenital disorders of glycosylation, and in particular PMM2 congenital disorder of glycosylation.

## Description

### Field of the Invention

The present invention relates to the medicinal use of small organic compounds, in particular amide and urea derivatives, for the treatment of congenital disorders of glycosylation.

### Background of the Invention

Glycosylation, the enzymatic attachment of carbohydrates (glycans) to proteins and lipids, plays a critical role in a variety of biological processes. Glycosylation defines protein folding and stability, and hence function, and also acts as a quality control mechanism that targets misfolded proteins for degradation. Glycan moieties may also act as ligands for cell surface receptors to mediate cell-cell adhesion, e.g. in immune responses, or stimulate signal transduction pathways.

Congenital disorders of glycosylation (CDGs), also known as CDG syndromes, are a group of rare genetic diseases wherein biosynthesis of glycoconjucates is deficient.

Common clinical symptoms in children with CDG include hypotonia, developmental delay, failure to thrive, hepatic dysfunction, coagulopathy, hypothyroidism, esotropia, abnormal fat pattern and inverted nipples, hypoglycemia, seizure, cerebellar hypoplasia, and stroke-like episodes in developmentally delayed children. At an older age, in adolescence, and adulthood, the presentation may include ataxia, cognitive impairment, and absence of puberty in females, small testes in males, retinitis pigmentosa, scoliosis, joint contractures, and peripheral neuropathy.

The most common congenital disorder of glycosylation is type la congenital disorder of glycosylation (*aka* CDG-Ia, PMM2-CDG, or CDGS-I) where mutation of the Phosphomannomutase 2 (PMM2) gene leads to non-functional or sub-functional PMM2 enzyme. The PMM2 enzyme is a homodimeric, cytoplasmic protein that catalyzes the isomerization of mannose 6-phosphate to mannose 1-phosphate, a precursor to GDP-mannose. GDP-mannose is necessary for the synthesis of dolichol-P-oligosaccharides, which play an important role in *N*-linked glycosylation and thus in a number of crucial biological pathways.

PMM2-CDG is a multisystemic disorder which can lead to very variable phenotypes and degrees of seriousness. It is mostly a severe disorder which presents neonatally. Encephalopathy with axial hypotonia, abnormal eye movements and pronounced psychomotor retardation, as well as a peripheral neuropathy, cerebellar hypoplasia and retinitis pigmentosa usually underlie the disorder. Patients also tend to show a peculiar distribution of subcutaneous fat, nipple retraction and hypogonadism. There is a 20% lethality in the first years of life due to severe infections, liver insufficiency or cardiomyopathy.

Currently, treatment of the vast majority of CDGs is limited to controlling some of the symptoms as opposed to targeting the disease itself. Examples include nutritional supplements, tube feeding or intravenous hydration and physical therapy for stroke-like episodes. Adults with orthopedic symptoms often require wheel chairs, transfer devices, and surgical treatment for scoliosis. Unfortunately, the severity or nature of many CDG symptoms renders them uncontrollable.

MPI-CDG (type Ib CDG) is the only known CDG for which a relatively effective treatment is available, namely oral D-mannose administration.

In the case of PMM2-CDG, whilst D-mannose administration is suggested in the literature, reports on its usefulness are inconsistent (Kjaergaard et al., Acta. Paediatr., 1998, 87(8), 884-888).

Another therapeutic strategy which has been proposed in the art for the treatment of PMM2-CDG is the inhibition of phosphomannose isomerase (MPI or PMI). MPI converts mannose 6-phosphate (as mentioned above also a substrate of the PMM2 enzyme) into fructose 6-phosphate. It has been suggested that inhibition of MPI can increase the mannose 6-phosphate pool and thus promote conversion of mannose 6-phosphate into mannose 1-phosphate for glycosylation (Sharma et al., J. Biol. Chem., 2011, 286(45), 39431-39438). However, such a therapeutic strategy is not optimal since it requires hindering the metabolic function of MPI, which itself is involved in important biosynthetic pathways such as glycolysis or capsular polysaccharide biosynthesis.

Gene therapy has also been suggested for restoring functional PMM2 protein (Vega, et al., Hum Mutat, 2009, 30(5), 795-803). However, whilst it has indeed proven useful in specific contexts, gene therapy is at the practical level generally subject to greater restrictions than standard small molecule drug therapy, both from a cost and efficiency point of view.

It is thus evident that the problem of treating congenital disorders of glycosylation, and in particular PMM2-CDG, is still far from being satisfactorily resolved.

Therefore, given the potential severity and even mortality of the diseases, an urgent need exists to develop effective treatments for congenital disorders of glycosylation, and in particular for PMM2-CDG.

Furthermore, treatments should ideally be based on small organic compounds, rather than on more complex, costly treatments which may in real practice be poorly effective and/or prohibitive, and should also ideally not involve altering fundamental biochemical pathways.

### Summary of the Invention

It has now surprisingly been found that particular small organic compounds are useful in the treatment of congenital disorders of glycosylation.

Thus, the present invention relates to a compound of formula (I) 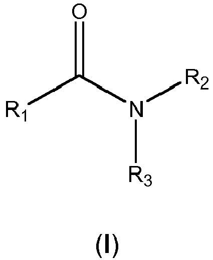 wherein
- R₁: is
- a phenyl ring, or a 6- or 5-membered heterocyclic ring containing one or more heteroatoms selected from O, N and S; wherein the rings are unsubstituted or substituted at one or more available positions with NH₂, OH, halogen, phenyl, C₁-C₆ alkyl (preferably methyl), -O-C₁-C₆ alkyl (preferably OMe), =O, or =NH;
- a fused ring group consisting of 2 or 3 rings, wherein each ring is independently a phenyl ring or a 6- or 5-membered heterocyclic ring containing one or more heteroatoms selected from O, N and S; and wherein each ring is independently unsubstituted or substituted at one or more available positions with NH₂, OH, halogen, phenyl, C₁-C₆ alkyl (preferably methyl), -O-C₁-C₆ alkyl (preferably OMe), =O, or =NH;
- NHR₄, wherein R₄ is a phenyl ring, or a 6- or 5-membered heterocyclic ring containing one or more heteroatoms selected from O, N and S; and wherein the rings are unsubstituted or substituted at one or more available positions with NH₂, OH, halogen, phenyl, C₁-C₆ alkyl (preferably methyl), -O-C₁-C₆ alkyl (preferably OMe), =O, or =NH; or
- -CH₂-R₅, wherein R₅ is -CN, or a phenyl ring, or a 6- or 5-membered heterocyclic ring containing one or more heteroatoms selected from O, N and S; and wherein the rings are unsubstituted or substituted at one or more available positions with NH₂, OH, halogen, phenyl, C₁-C₆ alkyl (preferably methyl), -O-C₁-C₆ alkyl (preferably OMe), =O, or =NH;
- R₂: is -(CH₂)ₙ-X, wherein n is 0, 1, 2 or 3, and X is
- a phenyl ring, or a 6- or 5-membered heterocyclic ring containing one or more heteroatoms selected from O, N and S; and wherein the rings are unsubstituted or substituted at one or more available positions with NH₂, OH, halogen, CF₃, C₁-C₆ alkyl (preferably methyl), -O-C₁-C₆ alkyl (preferably OMe), =O, or - NHC(O)Me; or
- a fused ring group consisting of 2 rings, wherein each ring is independently a phenyl ring or a 6- or 5-membered heterocyclic ring containing one or more heteroatoms selected from O, N and S; and wherein each ring is independently unsubstituted or substituted at one or more available positions with NH₂, OH, halogen, CF₃, C₁-C₆ alkyl (preferably methyl), -O-C₁-C₆ alkyl (preferably OMe), =O, or -NHC(O)Me;
- R₃: is H or -(CH₂)ₙ-X, wherein X and n are as defined for R₂;
or R₂ and R₃, together to the nitrogen atom to which they are bound, form a pyrrolidine
or piperidine ring;
or a pharmaceutically acceptable salt or solvate thereof;
for use as a medicament.

More particularly, the present invention relates to a compound of formula (I) or a pharmaceutically acceptable salt or solvate thereof, for use in the treatment of a congenital disorder of glycosylation.

More particularly, the present invention relates to a compound of formula (I) or a pharmaceutically acceptable salt or solvate thereof, for use in the treatment of PMM2-CDG.

These aspects and preferred embodiments thereof are also additionally defined herein below in the detailed description and in the claims.

### Brief description of the drawings

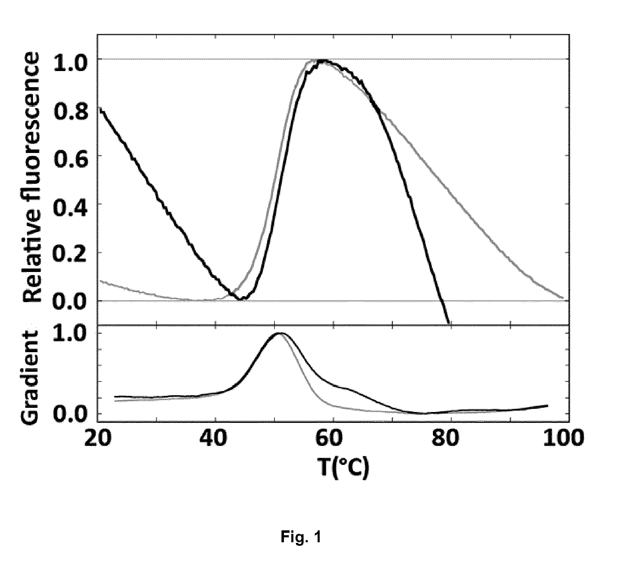

**Figure 1****.** Differential scanning fluorimetry (DSF) profile of Compound A obtained by high-throughput screening. Thermal denaturation profile of pure, homodimeric WT-PMM2 [fluorescence at 610 nm vs. temperature T (°C)], in the presence of 4% DMSO (grey line) and the compound at 2 mg/mL (black line). The experimental unfolding curves were then smoothed, normalized, and analysed using in-house software. The midpoint melting temperature (Tₘ) was calculated as that at which half of the protein was in the unfolded state. Difference in Tm (ΔTₘ) represents the difference between PMM2 Tₘ in the presence of DMSO and in presence of the compound. The underneath part of the graph represents the non-scaled curve. T(°C): temperature in Celsius.

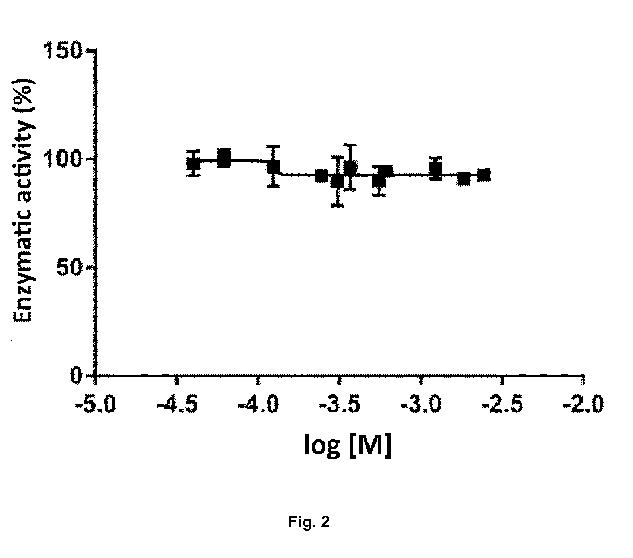

**Figure 2****.** Dose-response of pure WT-PMM2 protein incubated with Compound A. Representation of PMM2 activity (% respect to the protein incubated with DMSO) vs. logarithm of the compound concentration in Molarity [M].

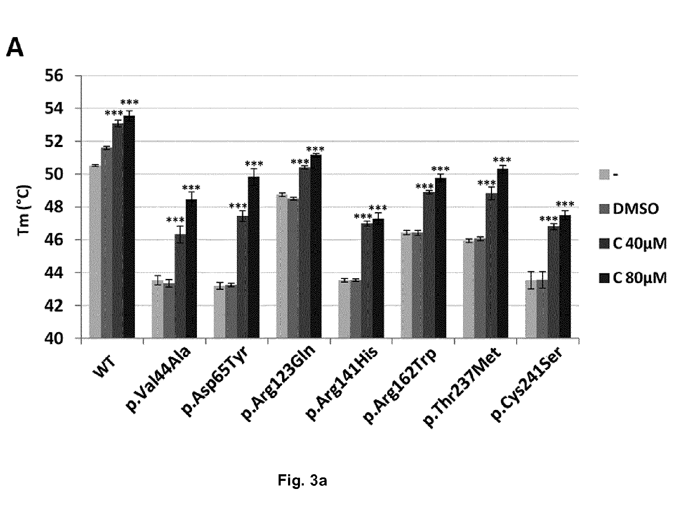

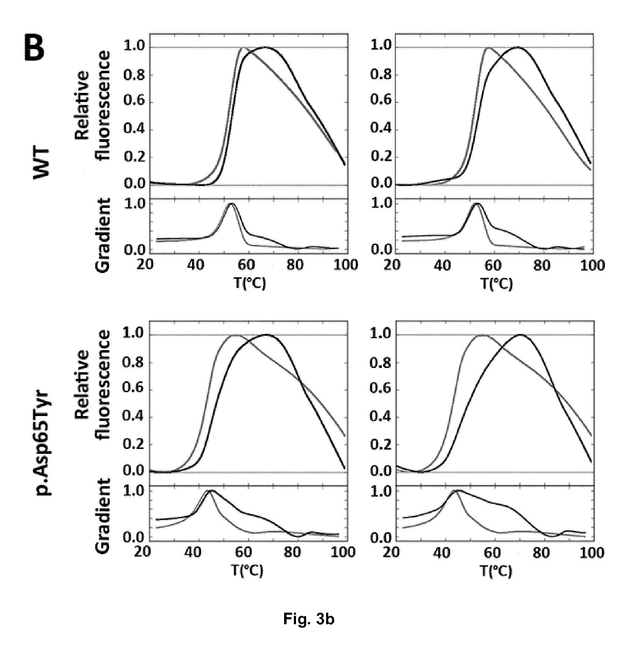

**Figure 3****.** Stabilizing effect of Compound A on the stability of WT-PMM2 and PMM2 mutants p.Val44AIa, p.Asp65Tyr, p.Arg123Gln, p.Arg141 His, p.Arg162Trp, p.Thr237Met and p.Cys241Ser, analyzed by DSF. A) *T*ₘ values from the DSF curves of the WT and mutant proteins incubated with the compound at 40µM and 80µM. The results are the average of at least three independent experiments. Significance was determined for each mutant respect itself incubated with DMSO ***p<0.001). B) Representative thermal denaturation profiles of WT-PMM2 and a characteristic mutant (caused by the mutation p.Asp65Tyr) incubated with DMSO (grey) and with the compound at 40 µM and 80 µM (black). T (°C): Temperature in Celsius.

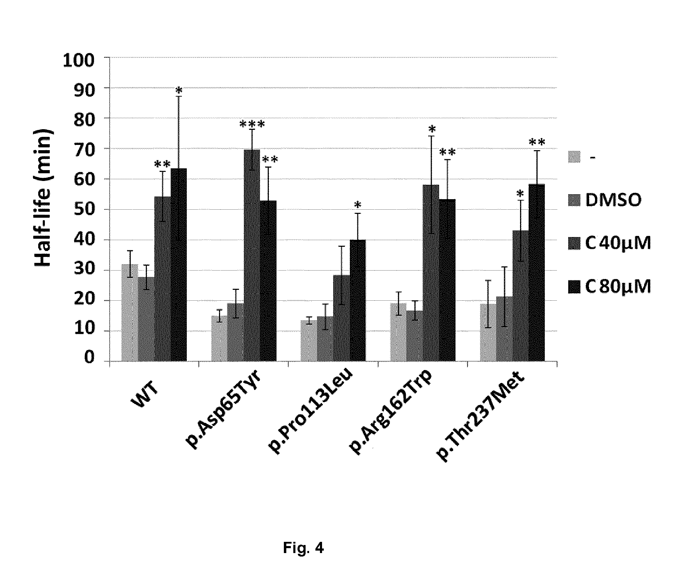

**Figure 4****.** Effect of Compound A on the stability of WT-PMM2 and PMM2 mutants (p.Asp65Tyr, p.Pro113Leu, p.Arg162Trp and p.Thr237Met), as determined by degradation time course analysis. Relative half-life (in minutes) of WT-PMM2 and mutant proteins in a prokaryotic transcription-translation-coupled system incubated with 40 µM or 80 µM of the compound. The results are the average of at least three independent experiments. Significance was determined by each mutant basal half-life (***p<0.001, **p<0.01, *p<0.05; compared to DMSO-controls).

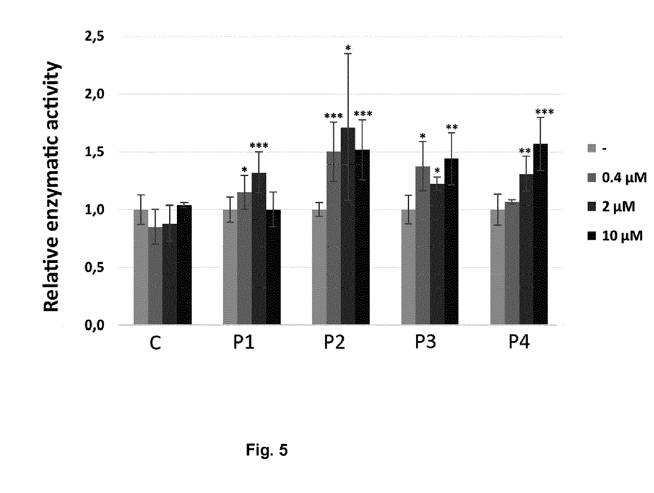

**Figure 5****.** Effect of Compound A on WT-PMM2 and mutant enzyme activity in the cellular disease model. Healthy and patient-derived fibroblasts stably transduced with the WT or their own folding mutation (p.Asp65Tyr, p.Pro113Leu, p.Arg162Trp and p.Thr237Met, respectively) were incubated with different concentrations of the compound for 48h. Baseline enzyme activity was considered as '1' in each cell line (-). Results are the mean ± SD of at least three independent experiments. Basal *p<0,05;**p<0,01; ***p<0,001 (differences in compound activity with respect to the corresponding baseline).

### Detailed Description of the Invention

In an embodiment, R₁ is as defined above but the option of R₅ being-CN is excluded.

In another embodiment, R₁ is
- a phenyl ring, or a 6- or 5-membered heteroaromatic ring containing one or two heteroatoms selected from O and N; and wherein the rings are unsubstituted or substituted at one or more available positions with halogen, -O-C₁-C₆ alkyl (preferably OMe) or C₁-C₆ alkyl, (preferably Me); or
- NHR₄, wherein R₄ is a phenyl ring, or a 6- or 5-membered heteroaromatic ring containing one or two heteroatoms selected from O and N; and wherein the rings are unsubstituted or substituted at one or more available positions with halogen, -O-C₁-C₆ alkyl (preferably OMe) or C₁-C₆ alkyl, (preferably Me).

In another embodiment, R₁ is a phenyl ring, or a 6- or 5-membered heteroaromatic ring containing one or two heteroatoms selected from O and N; and wherein the rings are unsubstituted or substituted at one or more available positions with halogen, -O-C₁-C₆ alkyl (preferably OMe) or C₁-C₆ alkyl, (preferably Me).

In another embodiment, R₁ is NHR₄, wherein R₄ is a phenyl ring, or a 6- or 5-membered heteroaromatic ring containing one or two heteroatoms selected from O and N; and wherein the rings are unsubstituted or substituted at one or more available positions with halogen, -O-C₁-C₆ alkyl (preferably OMe) or C₁-C₆ alkyl, (preferably Me).

In another embodiment, R₁ is
- a phenyl ring, or a 6-membered heteroaromatic ring containing one or two N heteroatoms; and wherein the rings are unsubstituted or substituted at one or more available positions with halogen; or
- NHR₄, wherein R₄ is a phenyl ring, or a 6-membered heteroaromatic ring containing one or two N heteroatoms; and wherein the rings are unsubstituted or substituted at one or more available positions with halogen.

In another embodiment, R₁ is a phenyl ring, or a 6-membered heteroaromatic ring containing one or two N heteroatoms; and wherein the rings are unsubstituted or substituted at one or more available positions with halogen.

In another embodiment, R₁ is NHR₄, wherein R₄ is a phenyl ring, or a 6-membered heteroaromatic ring containing one or two N heteroatoms; and wherein the rings are unsubstituted or substituted at one or more available positions with halogen.

Preferably, in any of the above embodiments, the heteroaromatic ring is pyridyl, even more preferably 2-pyridyl.

In a preferred embodiment, R₁ is
- a phenyl ring; wherein the ring is unsubstituted or substituted at one or more available positions with halogen; or
- NHR₄, wherein R₄ is a phenyl ring; wherein the ring is unsubstituted or substituted at one or more available positions with halogen.

In a preferred embodiment, R₁ is NHR₄, wherein R₄ is a phenyl ring, wherein the ring is unsubstituted or substituted at one or more available positions with halogen.

In another particular embodiment R₁ is a phenyl ring, wherein the ring is unsubstituted or substituted at one or more available positions with halogen.

Preferably, in any of the above embodiments, the halogen is Cl or F, more preferably it is Cl. Preferably, substitution is substitution at one or two positions, more preferably it is monosubtitution, even more preferably it is monosubtitution at the meta position. In a preferred embodiment, substitution is dichloro substitution, preferably 3,4-dichloro substitution; chloro-fluoro substitution, preferably 3-chloro-4-fluoro substitution; or chloro substitution, preferably 3-chloro substitution. Even more preferably, it is chloro substitution, preferably 3-chloro substitution.

In a particular embodiment, R₁ is one of the following:

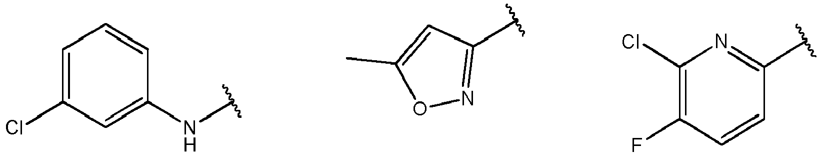

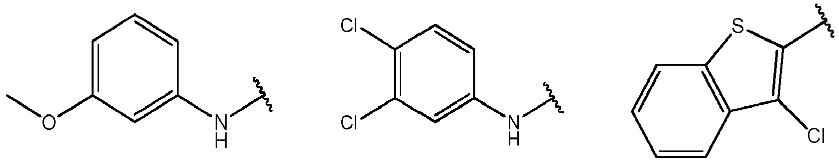

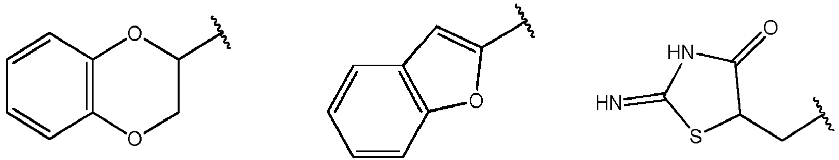

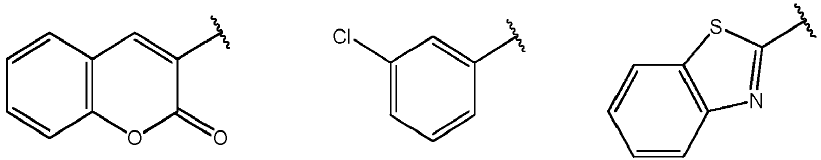

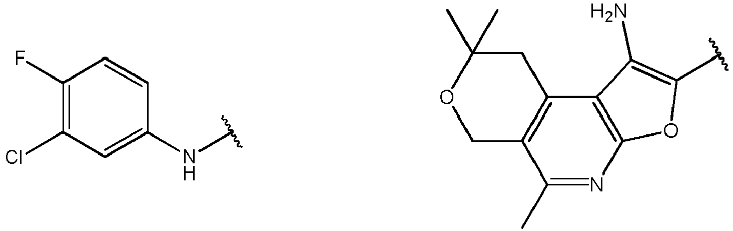

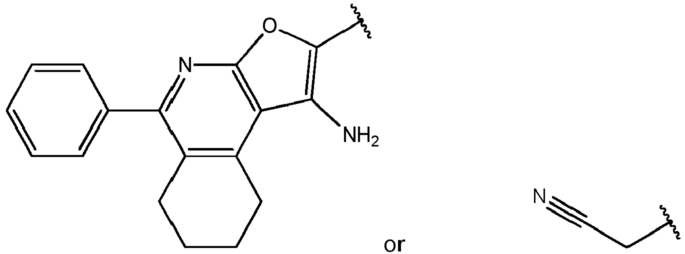

In a particular embodiment, R₁ is one of the following: 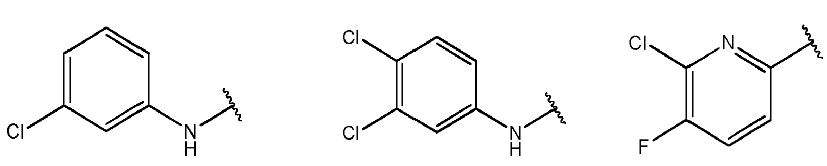 or

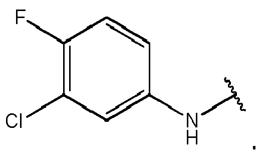

In a particular embodiment, R₁ is one of the following:

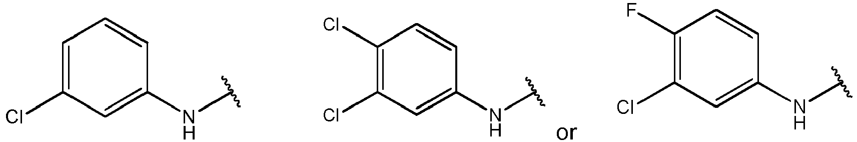

In a preferred embodiment, R₁ is

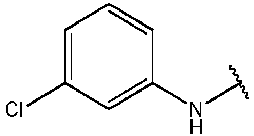

In an embodiment, R₂ and R₃ are as defined above, but the possibility of them forming a pyrrolidine or piperidine ring together to the nitrogen atom to which they are bound is excluded.
R₂ is a phenyl ring, or a 6- or 5-membered heteroaromatic ring containing one or more N heteroatoms; and wherein the rings are unsubstituted or substituted at one or more available positions with NH₂, OH, halogen or C₁-C₆ alkyl (preferably methyl); and
R₃ is H or a phenyl ring, or a 6- or 5-membered heteroaromatic ring containing one or more N heteroatoms; and wherein the ring is unsubstituted or substituted at one or more available positions with NH₂, OH, halogen or C₁-C₆ alkyl (preferably methyl).

In an embodiment, R₂ is a phenyl ring, or a 6- or 5-membered heteroaromatic ring containing one or more N heteroatoms; and wherein the rings are unsubstituted or substituted at one or more available positions with NH₂, OH, halogen or C₁-C₆ alkyl (preferably methyl).

In an embodiment, R₃ is H or a phenyl ring, or a 6- or 5-membered heteroaromatic ring containing one or more N heteroatoms; and wherein the ring is unsubstituted or substituted at one or more available positions with NH₂, OH, halogen or C₁-C₆ alkyl (preferably methyl).

In an embodiment, R₂ is phenyl or pyridyl; wherein the rings are unsubstituted or substituted at one or more available positions with NH₂, halogen or C₁-C₆ alkyl (preferably methyl); and
R₃ is H, or phenyl or pyridyl; wherein the rings are unsubstituted or substituted at one or more available positions with NH₂, halogen or C₁-C₆ alkyl (preferably methyl).

In an embodiment, R₂ is phenyl or pyridyl; wherein the rings are unsubstituted or substituted at one or more available positions with NH₂, halogen or C₁-C₆ alkyl (preferably methyl).

In an embodiment, R₃ is H, or phenyl or pyridyl; wherein the rings are unsubstituted or substituted at one or more available positions with NH₂, halogen or C₁-C₆ alkyl (preferably methyl).

Preferably, the halogen is Cl or F, more preferably it is Cl. Preferably, substitution is substitution at one or two positions, more preferably it is monosubtitution, even more preferably it is monosubtitution at the meta position, particularly preferably it is chloro substitution at the meta position.

Preferably, pyridyl is 2- or 3- pyridyl. Even more preferably, it is 2-pyridyl.

In any of the above embodiments, R₃ is preferably H. In a different embodiment, in any of the above embodiments R₂ and R₃ are the same.

In a particular embodiment, R₂ is one of the following, and/or R₃ is H or one of the following: 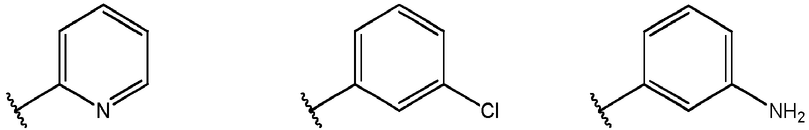 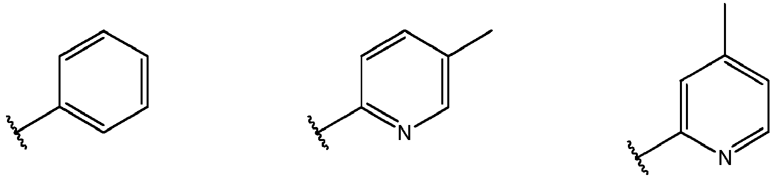 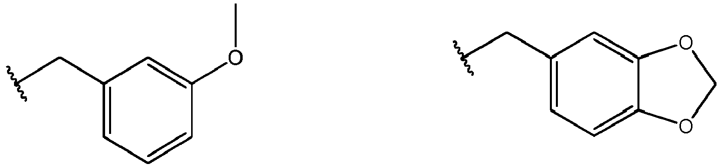 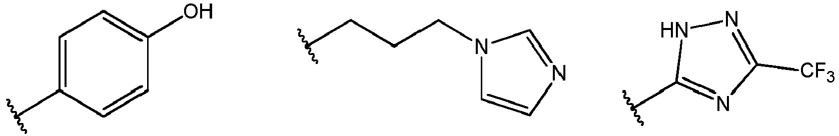 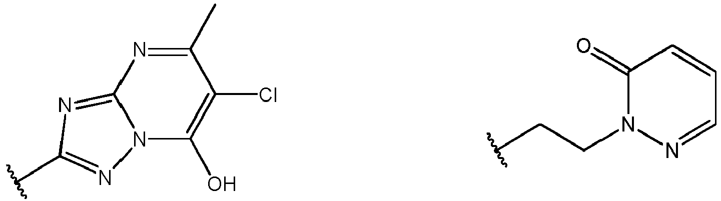 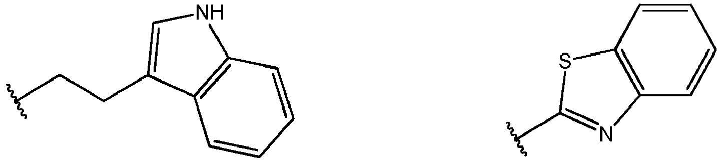 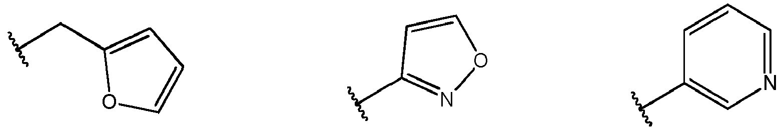 or

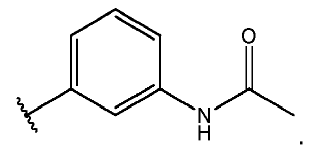

In a particular embodiment, R₂ is one of the following, and/or R₃ is H or one of the following: 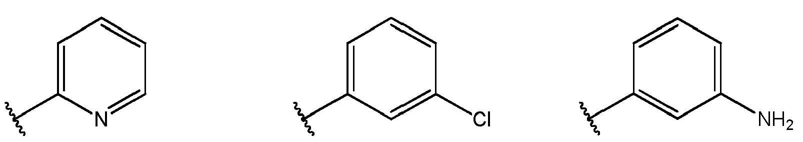 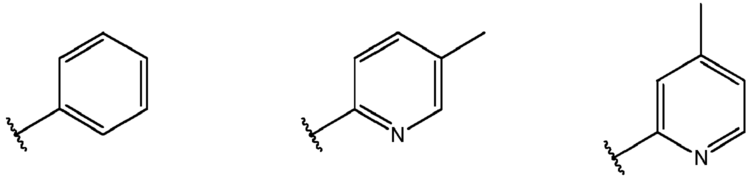 or

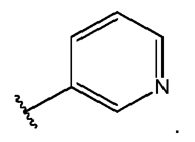

In a preferred embodiment, R₂ is one of the following, and/or R₃ is H or one of the following:

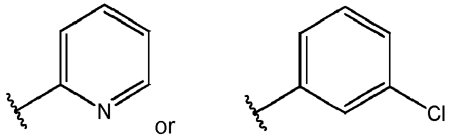

In a particular embodiment, 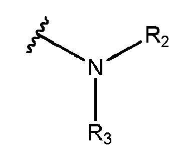 is

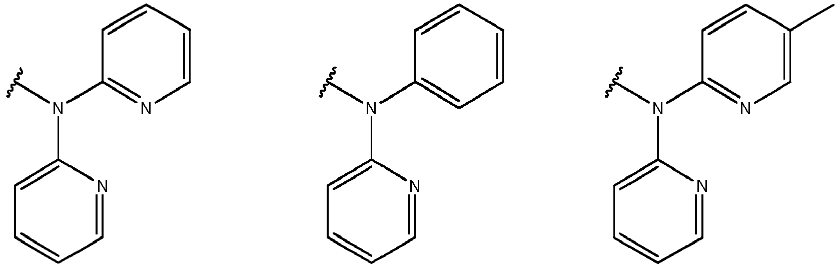

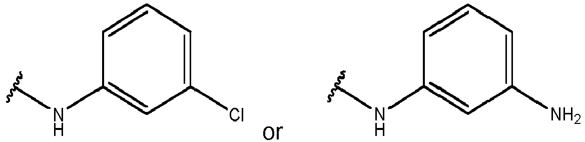

In a particular embodiment, 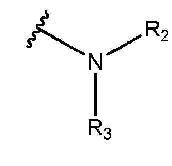 is

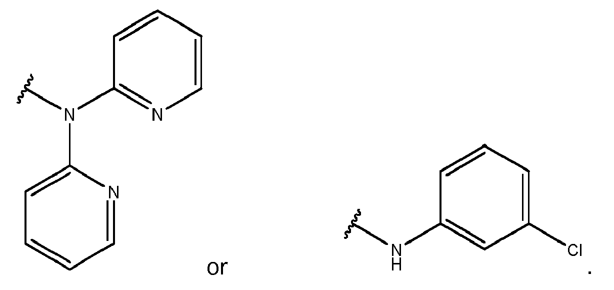

In a particular embodiment, the compound of formula (I) is one of the following: 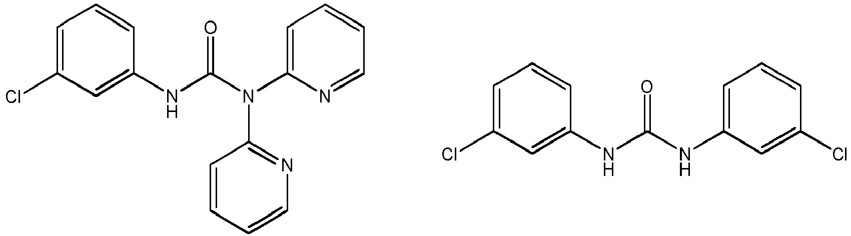 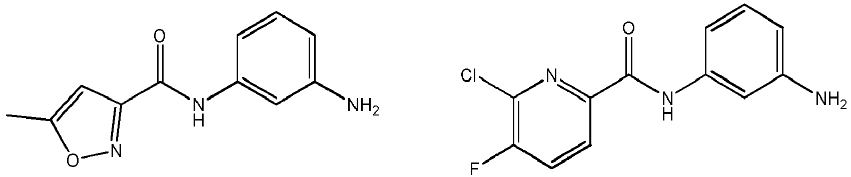 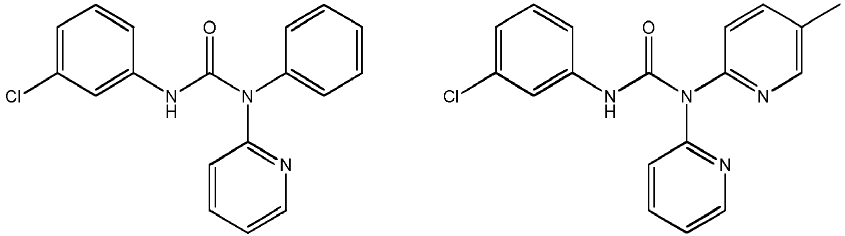 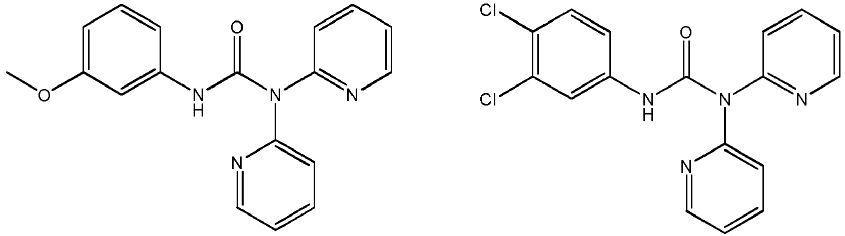 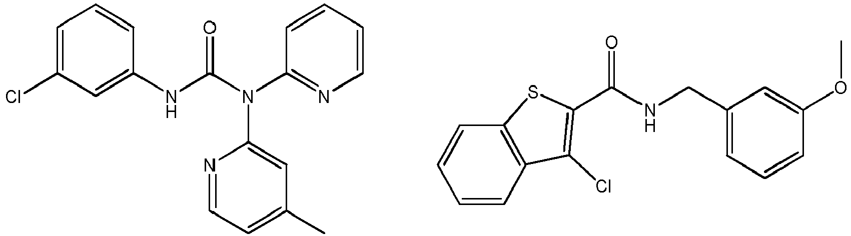 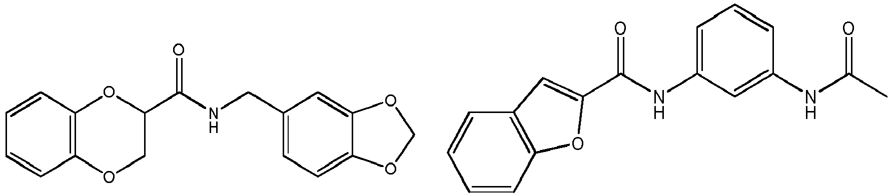 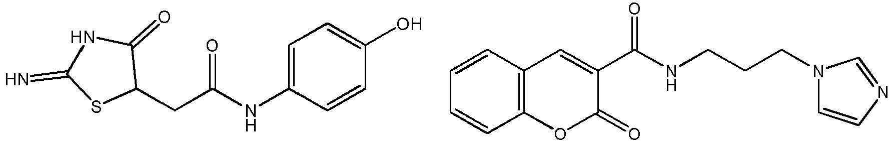 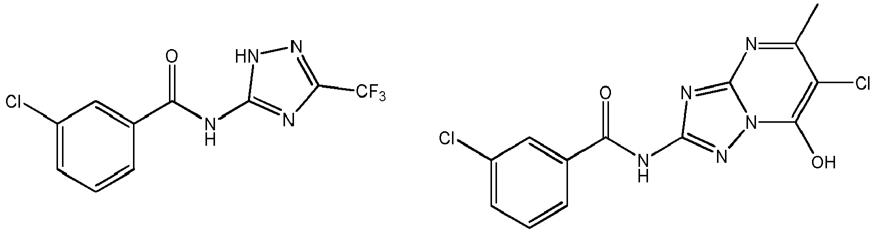 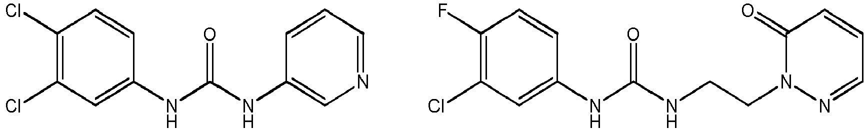 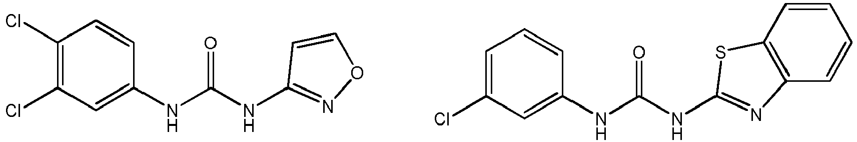 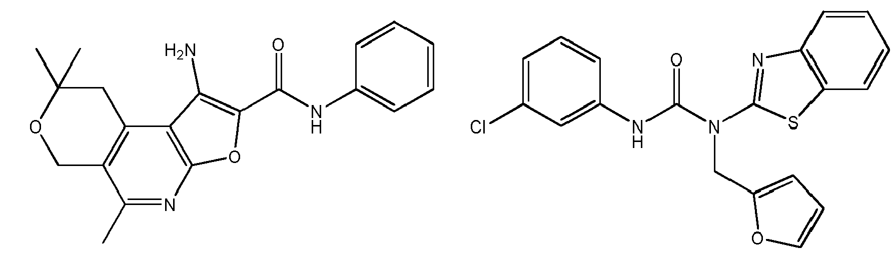 or

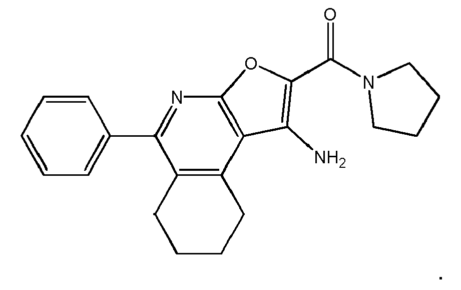

In a particular embodiment, the compound of formula (I) is one of the following:

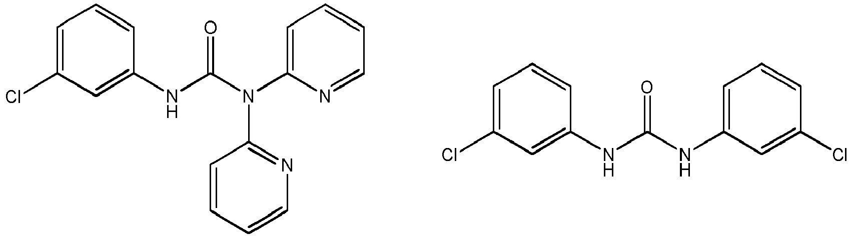

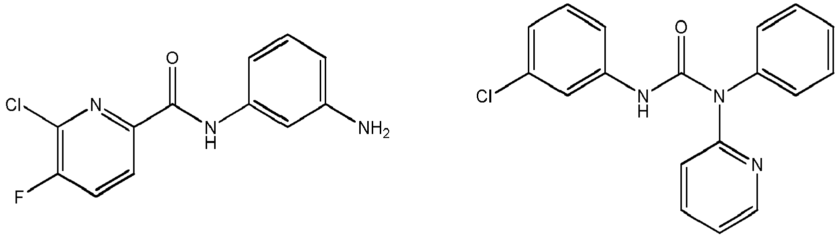

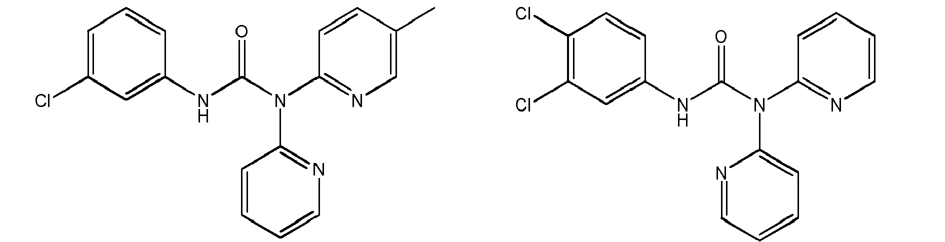

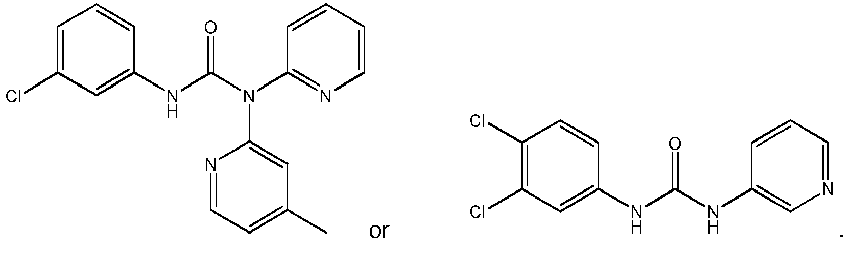

In a particular embodiment, the compound of formula (I) is one of the following:

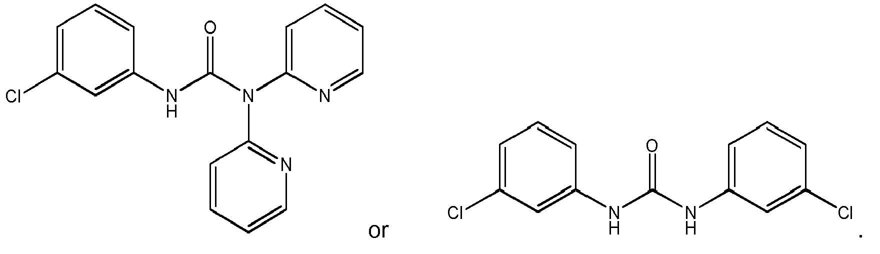

In a particular embodiment the compound of formula (I) is

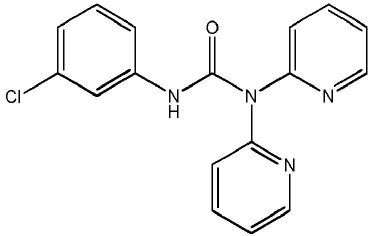

In another particular embodiment the compound of formula (I) is

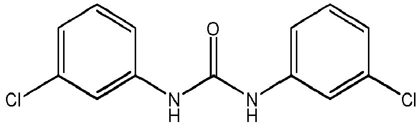

When in the above embodiments substitution is present at more than one available position, it is to be understood that each substituent at an available position is selected from the indicated list independently from substituents at other available positions, i.e. the substituent at one available position may be the same or different to the substituent at another available position.

In a particular embodiment, in any of the above embodiments which include a heteroaromatic ring containing any specified number or type of heteroatoms, this specification of number and type of heteroatom is limiting, i.e. the heteroaromatic ring cannot include any additional heteroatoms or types of heteroatoms other than those specified. For instance, where it is defined that a heteroaromatic ring contains one or two N heteroatoms, the ring cannot contain more than one or two heteroatoms and the heteroatoms cannot be other than N.

In the context of the present invention, "heterocyclic ring" refers to a stable ring radical which consists of carbon atoms and from one to five heteroatoms. The term "heterocyclic ring" includes according to the present invention both heteroaromatic and heteroalicyclic groups. In a preferred embodiment, the heterocyclic ring is a heteroaromatic ring. Examples of heteroaromatic groups include pyridyl, pyrazinyl, pyrazolyl, pyrimidinyl, furyl, pyrrolyl, thienyl, thiazolyl, isothiazolyl, triazolyl, tetrazolyl, isoxazolyl, oxazolyl. Examples of heteroalicyclic groups include pyrrolidinyl, tetrahydrofuryl, dihydrofuryl, tetrahydrothienyl, tetrahydrothiopyranyl, piperidyl, morpholinyl, thiomorpholinyl, piperazinyl.

In the context of the present invention, "alkyl" refers to a radical having a linear or branched hydrocarbon chain, containing no unsaturation, and binding to the rest of the molecule by means of a single bond. C₁-C₆ alkyl radicals have from 1 to 6 carbon atoms. In some embodiments, the alkyl is a C₁-C₄ alkyl. In a preferred embodiment, the alkyl is methyl, ethyl, n-propyl, i-propyl, n-butyl, or t-butyl.

"Halogen" refers to bromine, chlorine, iodine or fluorine.

The term "salt" must be understood as any form of an active compound used according to this invention in which said compound is in ionic form or is charged and coupled to a counterion (a cation or anion).

In the context of this invention, the "pharmaceutically acceptable salt" means any salt that is tolerated at a physiological level (normally meaning that it is not toxic, particularly as the result of the counterion) when it is applied or used in a suitable manner for a treatment.

The preparation of pharmaceutically acceptable salts can be carried out by methods known in the art. For instance, pharmaceutically acceptable salts of compounds according to the invention can be prepared by reacting the free acid or base forms of these compounds with a stoichiometric amount of the appropriate base or acid in water or in an organic solvent or in a mixture of both. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, trifluoroacetate, maleate, fumarate, citrate, oxalate, succinate, tartrate, malate, mandelate, methanesulfonate and p-toluenesulfonate. Examples of the alkali addition salts include inorganic salts such as, for example, sodium, potassium, calcium and ammonium salts, and organic alkali salts such as, for example, ethylenediamine, ethanolamine, *N*,*N*-dialkylenethanolamine, triethanolamine and basic aminoacids salts.

According to this invention, the term "solvate" refers to any form of the compounds according to the invention in which said compound is bound by means of a noncovalent bond to another molecule (usually a polar solvent), particularly including hydrates and alcoholates, such as methanolate. A preferred solvate is the hydrate. Methods of solvation are well known in the art of the invention.

The compounds of the present invention can include stereoisomers depending on the presence of chiral centers. The use of enantiomers or diastereoisomers or mixtures thereof falls within the scope of the present invention. The use of geometrical isomers of the compounds of the invention also falls within the scope of the present invention.

Furthermore, compounds herein mentioned may exist in different tautomeric forms, the use of which also falls within the scope of the invention. Specifically, the term tautomer refers to one or two or more structural isomers of a compound that exist in equilibrium and are readily converted from one isomeric form to another. Examples are amine-imine, amide-imidic acid or keto-enol of tautomeric pairs.

The compounds of the invention are preferably in a pharmaceutically acceptable or substantially pure form. Pharmaceutically acceptable form implies that the compound has a pharmaceutically acceptable level of purity excluding normal pharmaceutical additives such as diluents and carriers, and that it does not include materials considered to be toxic at normal dosage levels. The purity levels for the pharmacological substance are preferably greater than 50%, more preferably greater than 70%, most preferably greater than 90%. In a preferred embodiment, it is greater than 95%.

The compounds of the invention are commercially available and/or can be prepared by conventional methods known in the state of the art. Thus, for example, the compounds of the invention are part of the *MyriaScreen Diversity Collection* library (Sigma-Aldrich). US 2857430 and Duarte et al., Green Process Synth, 2013, 2, 19-24 describe the synthesis of compounds according to the invention.

The invention also refers to a pharmaceutical composition comprising i) at least one compound of formula (I) (as described in any of the above embodiments) or a pharmaceutically acceptable salt or solvate thereof, and ii) at least one pharmaceutically acceptable excipient. It should be noted that reference to the compound of formula (I) "as described in any of the above embodiments" is only a reference to the structure of the compound and does not include purpose limitations recited in the referred-to embodiments.

Examples of pharmaceutical compositions include any solid composition (e.g. tablets, pills, capsules, granules) or liquid composition (e.g. solutions, suspensions or emulsions).

The compositions can for example be administered orally, topically, dermally, nasally, intravenously, intramuscularly, intraperitoneally, intracerobrospinally, intracranially, intraspinally, subcutaneously, intraarticularly, intrasynovialy, or intrathecaly. Other forms of administration are not excluded.

The term "excipient" refers to a natural or synthetic substance formulated alongside the active ingredient(s), included for the purpose of long-term stabilization, bulking up solid formulations that contain potent active ingredients (thus often referred to as "bulking agents", "fillers", or "diluents"), or to confer a therapeutic enhancement on the active ingredient in the final dosage form, such as facilitating drug absorption, reducing viscosity, or enhancing solubility. In a preferred embodiment, excipient is a carrier, adjuvant and/or vehicle. Carriers are forms in which substances for improving the administration and efficacy of drugs are incorporated. Drug carriers are used in drug delivery systems, such as controlled release technology for prolonging the actions of the drug *in vivo*, reducing the metabolism of the drug and reducing the toxicity of the drug. Carriers designed for increasing drug delivery to the target pharmacological action sites are also herein contemplated. An adjuvant is a substance added to a formulation of pharmacological products that affects the action of the drug substance in an expected manner. A vehicle is a substance, preferably without therapeutic action, used as a means for providing volume for medicinal product delivery. Such pharmaceutical carriers, adjuvants or vehicles can be sterile liquids, such as water and oils, including petroleum oils, or animal, vegetable or synthetic oils, such as peanut oil, soya bean oil, mineral oil, sesame seed oil and the like, excipients, disintegrants, wetting agents or diluents. Suitable pharmaceutical carriers are described in "Remington's Pharmaceutical Sciences" by E.W. Martin. The selection of these excipients and the amounts that will be used will depend on the application form of the pharmaceutical composition.

The present invention is directed to a compound of formula (I) as described in any of the above embodiments, or to a composition comprising a compound of formula (I) as described in any of the above embodiments, for use as a medicament.

More particularly, the present invention is directed to a compound of formula (I) as described in any of the above embodiments, or to a composition comprising a compound of formula (I) as described in any of the above embodiments, for use in the treatment of a congenital disorder of glycosylation.

More particularly, the present invention is directed to a compound of formula (I) as described in any of the above embodiments, or to a composition comprising a compound of formula (I) as described in any of the above embodiments, for use in the treatment of a type I congenital disorder of glycosylation.

More particularly, the present invention is directed to a compound of formula (I) as described in any of the above embodiments, or to a composition comprising a compound of formula (I) as described in any of the above embodiments, for use in the treatment of PMM2-CDG.

The present invention also refers to the use of a compound of formula (I) as described in any of the above embodiments in the manufacture of a medicament or pharmaceutical composition.

More particularly, the present invention also refers to the use of a compound of formula (I) as described in any of the above embodiments in the manufacture of a medicament or pharmaceutical composition for the treatment of a congenital disorder of glycosylation.

More particularly, the present invention also refers to the use of a compound of formula (I) as described in any of the above embodiments in the manufacture of a medicament or pharmaceutical composition for the treatment of a type I congenital disorder of glycosylation.

More particularly, the present invention also refers to the use of a compound of formula (I) as described in any of the above embodiments in the manufacture of a medicament or pharmaceutical composition for the treatment of PMM2-CDG.

The present invention also relates to a method for the treatment of a congenital disorder of glycosylation, the method comprising administering to the subject in need of such a treatment a therapeutically effective amount of a compound of formula (I).

More particularly, the present invention also relates to a method for the treatment of a type I congenital disorder of glycosylation, the method comprising administering to the subject in need of such a treatment a therapeutically effective amount of a compound of formula (I).

More particularly, the present invention also relates to a method for the treatment of PMM2-CDG, the method comprising administering to the subject in need of such a treatment a therapeutically effective amount of a compound of formula (I).

Type I CDGs are characterized by defects in the initial steps of *N*-linked protein glycosylation. Examples of type I CDGs include, without limitation, la (PMM2-CDG), Ib (MPI-CDG), Ic (ALG6-CDG), Id (ALG3-CDG), Ie (DPM1-CDG), If (MPDU1-CDG), Ig (ALG12-CDG), Ih (ALG8-CDG), Ii (ALG2-CDG), Ij (DPAGT1-CDG), Ik (ALG1-CDG), 1l (ALG9-CDG), Im (DOLK-CDG), In (RFT1-CDG), Io (DPM3-CDG), Ip (ALG11-CDG), Iq (SRD5A3-CDG), Ir (DDOST-CDG), DPM2-CDG, TUSC3-CDG, MAGT1-CDG or DHDDS-CDG.

In the context of the present invention, the amounts of the compounds of the invention or of the pharmaceutical compositions comprising the compounds of the invention administered to subjects suffering from a CDG, preferably PMM2-CDG, are therapeutically effective amounts.

A "therapeutically effective amount" is at least the minimum concentration required to effect a measurable improvement of disease, herein a CDG, preferably a type I CDG, more preferably PMM2-CDG. An effective amount can be provided in one or more administrations. A therapeutically effective amount herein may vary according to factors such as the disease state, age, sex, and weight of the subject, and the concentration at which any of the different compounds of the present invention elicits a desired response in the subject. A therapeutically effective amount is also one in which any toxic or detrimental effects of the compounds of the invention are outweighed by the therapeutically beneficial effects. The daily dosage for human beings can typically be in the range from 1 to 2000 milligrams, preferably from 1 to 1500 milligrams, more preferably from 1 to 1000 milligrams of drug substance that will be administered for one or several times a day.

As used herein, the terms "treat", "treating" and "treatment" generally include the eradication, elimination, reversal, alleviation or control of a CDG, preferably a type I CDG, more preferably PMM2-CDG, in a subject.

In the context of the invention, the term "subject" or "patient" includes any animal. In a particular embodiment, the animal is a vertebrate, preferably a mammal. Examples of mammals are mice, rats, horses, pigs, rabbits, cats, sheep, dogs, cows as well as human beings. In a preferred embodiment, the animal is a human being.

The compounds of this invention can be used individually or in combination with each other, or in combination or further combination with other drugs (combination therapy). The different active agents can be part of the same composition or can be provided as a separate composition for administration at the same time or at different times.

In a particular embodiment, at least two compounds according to the invention are used, optionally in combination with other drugs. In a more particular embodiment, two compounds according to the invention are used, optionally in combination with other drugs. In any of these embodiments, the two compounds are preferably

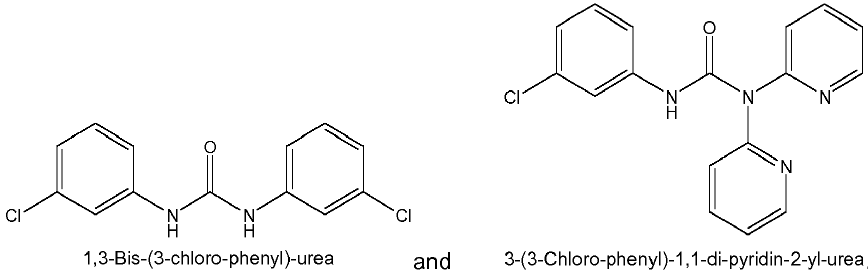

In an embodiment, when at least two compounds according to the invention are used, or when two compounds according to the invention are used, the weight proportion between the two compounds can be between 1:9 and 5:5, preferably between 2:8 and 4:6, more preferably between 3.5:6.5 and 2.5:7.5. In a particular embodiment the proportion is 3:7. In a preferred particular embodiment these ratios apply to 1,3-Bis-(3-chlorophenyl)-urea:3-(3-chlorophenyl)-1,1-di-pyridin-2-yl-urea.

In another particular embodiment, the compounds of the present invention are used in combination therapy with mannose 6-phosphate. In another particular embodiment, the compounds of the present invention are used in combination therapy with an inhibitor of MPI.

The present inventors have found that the compounds of formula (I) of the invention act as pharmacological chaperones to stabilise and enhance the activity of the phophomanomutase-2 enzyme. The chaperones of the invention can achieve this by improving enzyme specific activity (i.e. the amount of substrate an enzyme converts per milligram of protein in an enzyme preparation, per unit of time) and also by enhancing PMM2 protein half-life and thus PMM2 protein availability.

Furthermore, it has been unexpectedly observed that the stabilization and potentiation of enzymatic activity by the compounds of formula (I) of the invention is observed both for wildtype and mutated PMM2 enzymes. Advantageously, and even more surprisingly, the present inventors have demonstrated that stabilization and potentiation of enzymatic activity is achieved irrespective of the kind of PMM2 mutant. Indeed, as is demonstrated in the examples further below, the compounds of formula (I) of the invention are capable of acting as pharmacological chaperones of different kinds of mutants of PMM2 that are found in patients suffering from PMM2-CDG.

Thus, in an embodiment, treatment of a CDG, preferably a type I CDG, more preferably PMM2-CDG, is by stabilising PMM2 protein. In a particular embodiment, treatment of a CDG, preferably a type I CDG, more preferably PMM2-CDG, is by stabilising wildtype PMM2 protein. In another particular embodiment, treatment of a CDG, preferably a type I CDG, more preferably PMM2-CDG, is by stabilising mutant PMM2 protein. In another particular embodiment, treatment of a CDG, preferably a type I CDG, more preferably PMM2-CDG, is by stabilising mutant PMM2 protein as well as wildtype PMM2 protein.

In the context of the present invention, "stabilize", "stabilization" and related terms refer the ability of a pharmacological chaperone of the invention to induce a PMM2 conformation that is functionally closer, identical or superior to the conformation of the wildtype PMM2 protein that performs its intended function. In other words, it is not necessary that the stabilised conformation have all of the functional attributes of the wildtype protein. Many patients suffering from PMM2-CDG show 25% enzymatic PMM2 activity (100% being the activity observed in wildtype PMM2 homozygous subjects), whereas heterozygous subjects who retain 50% of normal PMM2 enzymatic activity are generally asymptomatic. It is thus clear that even small improvements in overall PMM2 enzymatic activity can have an enormous impact on the quality of life of subjects suffering from a CDG.

As used herein the term "mutant" refers to a PMM2 polypeptide translated from a PMM2 gene containing a genetic mutation that results in an altered PMM2 amino acid sequence which in turn leads to decreased stability or activity as compared to those of wild type PMM2 protein.

In an embodiment, the mutant results from at least one missense mutation in the PMM2 gene. In a particular embodiment, the mutation is in the coding region of the gene. In a particular embodiment, the mutant results from one missense mutation in the PMM2 gene. A missense mutation can also be referred to as a single point mutation.

In an embodiment, the PMM2 mutant is not totally enzymatically inactive. In another embodiment the PMM2 mutant possesses at least residual enzymatic activity. In an embodiment, residual enzymatic activity means that the mutant possesses at least 0.1%, at least 0.5%, or at least 1% enzymatic activity with respect to the enzymatic activity of the wildtype protein.

In a preferred embodiment, the mutation results in a PMM2 mutant protein that does not achieve a native (i.e. wild type) conformation. This type of mutation is referred to herein as a "conformational mutation," and the protein bearing such a mutation is referred as a "conformational mutant." The inability of the conformational mutant to achieve a native conformation leads to its decreased stability or activity. In an embodiment the CDG, preferably the type I CDG, more preferably PMM2-CDG, is fully or partially caused by said conformational mutant.

The term conformational mutant in the context of the present invention includes mutants which are unable to form the quaternary structure of native PMM2 protein. In a particular embodiment, the mutant is a dimerization mutant, i.e. a mutant which is unable to dimerize into the native structure of the PMM2 protein.

In a preferred embodiment, the mutant is a conformational mutant that results from at least one missense mutation in the PMM2 gene. In a particular embodiment, the mutant is a conformational mutant that results from one missense mutation in the PMM2 gene. In a particular embodiment, the mutation is in the coding region of the gene.

In a particular embodiment, the mutant is selected from the following list of mutants: p.Val44AIa, p.Asp65Tyr, p.Pro113Leu, p.Arg123Gln, p.Arg141His, p.Arg162Trp, p.Thr237Met, p.Cys241Ser. More particularly, the mutant is p.Val44AIa, p.Asp65Tyr, p.Pro113Leu, p.Arg123Gln, p.Arg162Trp, p.Thr237Met or p.Cys241Ser mutant. Even more particularly the mutant is p.Val44AIa, p.Asp65Tyr, p.Pro113Leu, p.Arg162Trp, p.Thr237Met or p.Cys241Ser mutant. In a preferred particular embodiment, the mutant is p.Asp65Tyr, p.Pro113Leu, p.Arg162Trp or p.Thr237Met mutant. In another particular embodiment the mutant is the dimerization mutant p.Pro113Leu.

It should be noted that the use of the compounds or compositions as described in any of the above embodiments in the treatment of a CDG, preferably a type I CDG, more preferably PMM2-CDG, is not limited to situations in which the CDG is caused by PMM2 conformational mutants. As mentioned above, the compounds of the invention stabilize even wildtype PMM2 enzyme. Thus, even where insufficient overall PMM2 enzymatic activity in a patient is not due to a misfolded PMM2 protein, the compounds and compositions of the invention may be used to potentiate the activity of properly folded PMM2 protein in order to thus increase overall PMM2 enzymatic activity. Therefore, in a particular embodiment, the CDG, preferably the type I CDG, more preferably PMM2-CDG, is not caused by a conformational mutant.

For instance, in a particular embodiment, the mutation is in a non-coding part of the PMM2 gene. This can result in less efficient expression of the protein, e.g., a mutation that affects transcription efficiency, splicing efficiency, mRNA stability, and the like. In such cases, PMM2 protein produced may have a native conformation or a mutated conformation which is considerably functional, however its reduced degree of expression results in overall insufficient PMM2 enzymatic activity.

The present inventors have further found that, as opposed to most pharmacological chaperones used in medicine, which act as competitive inhibitors of the natural substrate of the target protein, the compounds of formula (I) of the present invention do not interfere with PMM2 catalytic activity. Therefore, in one embodiment, the compounds of the invention are not competitive inhibitors. In another embodiment, the compounds of the present invention act allosterically on the PMM2 protein (mutated or wildtype).

The invention is described below by means of the following examples which must be considered as being merely illustrative and non-limiting thereof.

### EXAMPLES

### Example 1. High-throughput library screening by differential scanning fluorimetry.

10,000 molecular compounds from the MyriaScreen (Sigma-Aldrich) diversity collection were screened by determining the stabilization induced by the binding of different ligands (potential pharmacological chaperones) to the purified recombinant protein. The identification of ligands that stabilize WT-PMM2 thermally, was done by monitoring the thermal denaturation. The screening was performed by monitoring a shift in the fluorescence intensity at 610 nm using the LightCycler A480 (Roche).

PMM2 proteins were expressed in *E.coli* (BL21 Star™DE3 One Shot® Invitrogen). The expression plasmid pDEST17-18 encoding human *PMM2* (NM_000303.2) plus an N-terminal His₆-tag (Source BioScience, Nottingham, UK) was used to transform *E. coli* strain BL21Star™DE3 One Shot Cells (Invitrogen, Carlsbad, CA, USA). *PMM2* mutations were introduced by site-directed mutagenesis using the QuikChange Mutagenesis Kit (Stratagene, Cedar Drive, TX, USA) and specially designed primers. All products were verified by DNA sequencing. For protein expression, bacteria were grown in modified TYM medium (Studier Auto-Induction Medium with N-Z-amine replaced by tryptone) containing 25 µg/mL zeocine overnight at 37°C. Cells were harvested by centrifugation, resuspended in 20 mM Hepes, 25 mM KCI, 1 mM DTT, pH 7, and 1X Complete Mini, EDTA-free Protease Inhibitor Cocktail (Roche Applied Biosciences, Indianapolis, IN, US), lysed by sonication and then, centrifuged at 4°C.

Protein purification was performed using the ÄKTA Prime System (GE Healthcare, Little Chalfont, UK) at 4°C. The crude extract was loaded onto a HisTrap™ High Performance affinity column (GE Healthcare) equilibrated with 10 mM imidazole, 0.5 M NaCl, 20 mM sodium phosphate, pH 7.4, and eluted with an imidazole gradient from 10 mM to 1 M. The eluted protein fractions were pooled and loaded onto a Superdex 200 HiLoad 16/60 size exclusion chromatography column (GE Healthcare). The elution fraction corresponding to dimeric PMM2 was recovered and stored for further experiments. The pure protein concentration was estimated by measuring the absorbance at 280 nm in a Nanodrop spectrophotometer (Thermo Scientific, Waltham, MA, USA) using the theoretical molar extinction coefficient estimated from the amino acid composition of HisPMM2 (23755 M⁻¹ cm⁻¹).

10,000 compounds of molecular weight ranging from 220 to 547 Da from the MyriaScreen diversity collection, dissolved in DMSO (Sigma Aldrich, St. Louis, MO, USA) to a final concentration of 2 mg/mL, were screened by DSF for identification of ligands that stabilize WT-PMM2 thermally. This was done by monitoring the thermal denaturation in the presence of the extrinsic fluorescent probe SYPRO Orange (Sigma-Aldrich). Screening was performed by mixing 24 µl purified WT-PMM2 in HEPES-buffer with 1 µl compound in DMSO to a final concentration of 0.064 mg/mL (2.1 µM) WT-PMM2, 20 mM Na-Hepes pH 7.0, 200 mM NaCl, 0.08 mg/mL of compound (average concentration 200 µM), 4% DMSO (compound solvent) and 5x SYPRO Orange, in 384-well RT-PCR plates (Roche Applied Sciences). DMSO controls, with protein but without compounds, were included in each plate. The plates were loaded into a LightCycler480 (Roche Applied Science), and the thermal unfolding curves were recorded from 20 to 99°C at a scan rate of 2°C/minute by following the increase in SYPRO Orange fluorescence intensity associated with protein unfolding (λ_{excitation} = 465 nm, λₑₘᵢₛₛᵢₒₙ = 580 nm). The experimental unfolding curves were then smoothed, normalized, and analyzed using in-house software. The midpoint melting temperature (*T*ₘ) was calculated as the temperature for which half of the protein (at relative fluorescence 0.5) was in the unfolded state.

Compound selection for further testing was carried out based on the ability of the compound to confer resistance to the thermal denaturation of the protein. Figure 1 shows the DSF curve for Compound A. As can be seen, protein Tₘ is increased 1.1 °C in the presence of Compound A. Compound A was found to be 3-(3-Chloro-phenyl)-1,1-di-pyridin-2-yl-urea with 1,3-Bis-(3-chloro-phenyl)-urea as an impurity.

### Example 2. Inhibitory effect of Compound A on the pure WT-PMM2

Given that the description of most of the described pharmacological chaperones has a competitive inhibitory effect for the target enzyme, we decided to analyze Compound A's IC₅₀.

For the calculation of IC₅₀ values, pure WT-PMM2 was used. Fifty nanograms of Nanodrop-measured pure dimeric PMM2 (molar extinction coefficient 23755 M⁻¹ cm⁻¹) were incubated with 1 µl of the compound at different concentrations, along with 0.5% BSA for 10 min at 30°C. After that time, the activity assays were performed by a spectrophotometric method based on the NADPH determination at 340 nm produced by a chain reaction of different coupled enzymes and the final production of NADP by Glucose-6P-dehydrogenase enzyme (de Koning et al., 1998; Van Schaftingen y Jaeken, 1995). The corresponding DMSO controls were included in the assay. The IC₅₀ values for the inhibitory compound were calculated using non-linear regression (GraphPad Prism 6 software) by plotting the log concentration of the compound (M) versus WT-PMM2 enzyme activity (%).

Results showed that the compound has no inhibitory effect on the activity of the pure WT-PMM2 protein at the tested concentrations (2.5 mM was the maximum concentration used) (Figure 2).

### Example 3. Effect of Compound A on the stability of WT-PMM2 and mutants by DSF

The effect of Compound A on the stability of different PMM2 folding mutants was studied by DSF. The selected mutants were the folding mutants, p.Val44AIa, p.Asp65Tyr, p.Arg162Trp, p.Thr237Met and p.Cys241Ser and the catalytic and folding mutants, p.Arg123Gln and p.Arg141His as previously described. The effect was studied at 40 µM and 80 µM compound concentration over the thermal stability of the purified homodimeric mutants by DSF.

In this case, the DSF was performed in a final reaction volume of 50 µl, containing 0.075 mg/ml of pure PMM2 protein in Hepes 20 mM, NaCl 200 mM, SYPRO Orange 5X, 1 µl of the hit compound and 2% DMSO in a LightCycler Multiwell Plate 96 (Roche).

Results showed a positive and statistically significant ΔTₘ, between 1.5°C and 6.5°C,of all the unstable mutants included in the assay incubated with the compound at 40 µM and 80 µM. These results indicate that the stabilizing effect is not mutant-specific.

Moreover, it is remarkable that the p.Arg123Gln mutant at both concentrations reached the WT-PMM2 basal Tm, like the p.Asp65Tyr and p.Thr237Met mutants incubated with 80µM of the compound (Figure 3).

### Example 4. Evaluation of the effect of Compound A on WT and PMM2 mutant stability by degradation time course

In order to deepen in the effect of Compound A on the PMM2 stability, degradation time courses were analyzed on WT protein and folding mutants with residual activity, p.Asp65Tyr, p.Arg162Trp and p.Thr237Met in presence of the compound. Additionally, we included in this assay the dimeric mutant p.Pro113Leu in order to study the effect of the compound on a mutant of this type.

Dimeric PMM2 used for thermal stability analysis was produced by the RTS 100 *E. coli* HY Kit transcription-translation-coupled system (5 PRIME, Hilden, Germany), following the manufacturer's recommendations. To produce PMM2 proteins, the reaction mix provided by the kit, together with 500 ng of the corresponding expression vector and 1 µl of the compound for reaching a final concentration of 40µM or 80µM or 1µl of DMSO. Reactions were incubated for 30 min at 30°C. The reaction was then stopped by adding 1 µl of DNase (1 mg/mL) and 1 µl of RNase (1 mg/mL). It was then further incubated at 37°C and 1.5 µl aliquots removed at different times. The compound was therefore present during the folding process. Proteins were immunodetected by Western blot and relative amounts of protein were determined by densitometry for the half-life determination.

Results showed a significant half-life increase for the WT-PMM2 and the four mutants with respect their basal half-lives (with DMSO) at, at least, one of the tested concentrations. Increases ranged from 1.7 to 3.5 times higher than the basal half-life (with DMSO), being the combination of p.Asp65Tyr with the compound at 80µM the one with the higher shift (Figure 4).

It is important to note that the increase in the half-life of the mutant proteins, at least at the tested concentrations, afforded them a longer half-life than that recorded for the WT-PMM2 (Figure 4).

### Example 5. Cellular model used to examine PMM2 activity in the presence of the Compound A

Finally, it was analyzed the effect of Compound A on a cellular model of disease. Due to the low expression of PMM2 protein in fibroblasts, we generated a cellular model using fibroblast lines from control lines or derived from patients diagnosed as PMM2-CDG, P1 (p.Arg141 His/p.Asp65Tyr), P2 (p.Pro113Leu/p.Pro113Leu), P3 (p.Arg141His/p.Arg162Trp) and P4 (c.640-9T>G/p.Thr237Met), that were transduced with their WT, own folding or oligomerization mutation, (WT, p.Asp65Tyr, p.Pro113Leu, p.Arg162Trp y p.Thr237Met, respectively).

The full-length ORF of human *PMM2* was cloned into the mammalian lentiviral plasmid pReceiver-Lv101 (EX-M0134-Lv101) (GeneCopoeia, Rockville, MD, USA), which contains the FLAG tag preceding the multiple cloning site. Lentiviral stock production and fibroblast infection were performed harvesting 4,5x10⁶ cells HEK-293T in p100 plate transfected with Lipofectamine (Life Teachnologies). pCMv-dR8.74 vector was used as lentiviral packaging vector and pMD2.G was used for the enveloped proteins. Efficiently infected fibroblasts were selected by Geneticin treatment at 500 µg/mL. GM08680 cellular line (Coriell Institute for Medical Research, NIGMS Human Genetic Cell Repository, Camden, NJ) was used as control. The cellular lines were previously immortalized using the pBABE-pure vector containing SV40 LT cDNA.

These cell lines were then incubated with the compound at 0.4µM, 2µM and 10µM for 48 hours, and then the activity assay was performed. Cell viability was previously confirmed to be high at these concentrations. The activity assay was performed as described before for the pure PMM2 protein, using the method of Van Schaftingen and Jaeke 1995, as modified by Koning *et al.* 1998, butadding some modifications for increasing the yield and sensitivity of the technique. These modifications included the use of a commercial lysis buffer and the amplification of the signal of the enzymatic reaction product, NADPH. 1.5x10⁴ fibroblast from control lines or derived from patients overexpressing the protein were seeded in a p96 plate. After treatment, cells were lysated directly on the plate using the Complete Lysis M (Roche) buffer. Reaction mixture was added directly to the p96 plate and it was incubated at 37°C during 45 minutes. After that, the reaction was stopped as described in the classical method, and the NADPH detection was amplified using Amplite Fluorimetric NADPH assay Kit Red Fluorescence (AAT Bioquest). Data was obtained reading the signal at 540nm/590nm in a FLUOstar OPTIMA (BMG LABTECH).

An increase in enzyme activity was achieved with the compound in patient-derived cells overexpressing the mutant proteins p.Asp65Tyr, p.Pro113Leu, p.Arg162Trp and p.Thr237Met; the increase ranged from 1.15 to 1.7 times the baseline activity recorded for each cell line respectively (Figure 5). The greatest recovery observed was in P2 cell line overexpressing the p.Pro113Leu mutation incubated with 2µM of the compound (Figure 5).

The greatest effect showed in the case of the cell lines overexpressing p.Asp65Tyr and p.Pro113Leu mutant proteins was observed at 2 µM of the compound, producing an increment from a cell line baseline value of about 28% (WT activity representing 100%), to about 37% in the presence of Compound A, and from about 20% to about 32%, respectively.

For cell lines overexpressing p.Arg162Trpr and p.Thr237Met mutants, the highest increase was observed at 10µM, from about 13% of baseline activity to about 19% and from about 53% to about 84% respectively (Table 1).

**Table 1 shows the activity values for the WT-PMM2 and mutant PMM2 cell lines (healthy and patient-derived fibroblast overexpressing WT protein and the p.Asp65Tyr, p.Pro113Leu, p.Arg162Trp and p.Thr237Met unstable mutations, respectively), incubated for 48 h. Comparisons were made with baseline activity of the WT-PMM2 cell line (taken as 100%).**

| | | **Control (WT)** | **P1 (p.Asp65Tyr)** | **P2 (p.Pro113Leu)** | **P3 (p.Arg162Trp)** | **P4 (p.Thr237Met)** |
|---|---|---|---|---|---|---|
| | | 100±1.7 | 27.9±1.8 | 19±8 | 13.3±2.8 | 53.4±8.6 |
| **Compound** | **0.4µM** | 85±15 | 32.1 ±4.2* | 28.5±4.9^{***} | 18.2±2.8^{*} | 56.6±1.1 |
| | **2µM** | 88±15.5 | 36.8±5^{***} | 32.5±12.2^{*} | 16.2±0.8^{*} | 69.9±8^{**} |
| | **10µM** | 104±2.1 | 27.9±4.2 | 28.9±4.9^{***} | 19.1±3^{**} | 83.8±12.3^{**} |

| | | | | | | |
|---|---|---|---|---|---|---|
| Results represent the mean ± SD of at least three independent experiments. Statistical significance of each mutant respect to the corresponding baseline (^{*}p<0.05;^{**}p<0.01; ^{***}p<0.001). | | | | | | |

## Claims

1. Compound of formula (I) 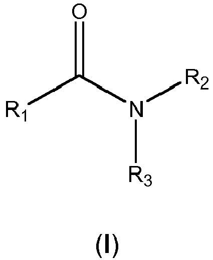 wherein
R₁ is
- a phenyl ring, or a 6- or 5-membered heterocyclic ring containing one or more heteroatoms selected from O, N and S; wherein the rings are unsubstituted or substituted at one or more available positions with NH₂, OH, halogen, phenyl, C₁-C₆ alkyl (preferably methyl), -O-C₁-C₆ alkyl (preferably OMe), =O, or =NH;
- a fused ring group consisting of 2 or 3 rings, wherein each ring is independently a phenyl ring or a 6- or 5-membered heterocyclic ring containing one or more heteroatoms selected from O, N and S; and wherein each ring is independently unsubstituted or substituted at one or more available positions with NH₂, OH, halogen, phenyl, C₁-C₆ alkyl (preferably methyl), -O-C₁-C₆ alkyl (preferably OMe), =O, or =NH;
- NHR₄, wherein R₄ is a phenyl ring, or a 6- or 5-membered heterocyclic ring containing one or more heteroatoms selected from O, N and S; and wherein the rings are unsubstituted or substituted at one or more available positions with NH₂, OH, halogen, phenyl, C₁-C₆ alkyl (preferably methyl), -O-C₁-C₆ alkyl (preferably OMe), =O, or =NH; or
- -CH₂-R₅, wherein R₅ is -CN, or a phenyl ring, or a 6- or 5-membered heterocyclic ring containing one or more heteroatoms selected from O, N and S; and wherein the rings are unsubstituted or substituted at one or more available positions with NH₂, OH, halogen, phenyl, C₁-C₆ alkyl (preferably methyl), -O-C₁-C₆ alkyl (preferably OMe), =O, or =NH;
R₂ is -(CH₂)ₙ-X, wherein n is 0, 1, 2 or 3, and X is
- a phenyl ring, or a 6- or 5-membered heteroaromatic ring containing one or more heteroatoms selected from O, N and S; and wherein the rings are unsubstituted or substituted at one or more available positions with NH₂, OH, halogen, CF₃, C₁-C₆ alkyl (preferably methyl), -O-C₁-C₆ alkyl (preferably OMe), =O, or -NHC(O)Me; or
- a fused ring group consisting of 2 rings, wherein each ring is independently a phenyl ring or a 6- or 5-membered heteroaromatic ring containing one or more heteroatoms selected from O, N and S; and wherein each ring is independently unsubstituted or substituted at one or more available positions with NH₂, OH, halogen, CF₃, C₁-C₆ alkyl (preferably methyl), -O-C₁-C₆ alkyl (preferably OMe), =O, or -NHC(O)Me;
R₃ is H or -(CH₂)ₙ-X, wherein X and n are as defined for R₂;
or R₂ and R₃, together to the nitrogen atom to which they are bound, form a pyrrolidine or piperidine ring;
or a pharmaceutically acceptable salt or solvate thereof;
for use as a medicament.

2. Compound, salt or solvate for use according to claim 1, wherein R₁ is
- a phenyl ring, or a 6- or 5-membered heteroaromatic ring containing one or two heteroatoms selected from O and N; and wherein the rings are unsubstituted or substituted at one or more available positions with halogen, -O-C₁-C₆ alkyl (preferably OMe) or C₁-C₆ alkyl, (preferably Me); or
- NHR₄, wherein R₄ is a phenyl ring, or a 6- or 5-membered heteroaromatic ring containing one or two heteroatoms selected from O and N; and wherein the rings are unsubstituted or substituted at one or more available positions with halogen, -O-C₁-C₆ alkyl (preferably OMe) or C₁-C₆ alkyl, (preferably Me).

3. Compound, salt or solvate for use according to any preceding claim, wherein R₁ is a phenyl ring, or a 6-membered heteroaromatic ring containing one or two N heteroatoms; and wherein the rings are unsubstituted or substituted at one or more available positions with halogen.

4. Compound, salt or solvate for use according to any preceding claim, wherein R₁ is a phenyl ring; wherein the ring is unsubstituted or substituted at one or more available positions with halogen.

5. Compound, salt or solvate for use according to claims 1 or 2, wherein R₁ is NHR₄, wherein R₄ is a phenyl ring, or a 6-membered heteroaromatic ring containing one or two N heteroatoms; and wherein the rings are unsubstituted or substituted at one or more available positions with halogen.

6. Compound, salt or solvate for use according to claims 1-2 or 5, wherein R₁ is NHR₄, wherein R₄ is a phenyl ring; wherein the ring is unsubstituted or substituted at one or more available positions with halogen.

7. Compound, salt or solvate for use according to any preceding claim, wherein R₂ is a phenyl ring, or a 6- or 5-membered heteroaromatic ring containing one or more N heteroatoms; and wherein the rings are unsubstituted or substituted at one or more available positions with NH₂, OH, halogen or C₁-C₆ alkyl.

8. Compound, salt or solvate for use according to any preceding claim wherein R₂ is phenyl or pyridyl; wherein the rings are unsubstituted or substituted at one or more available positions with NH₂, halogen or C₁-C₆ alkyl.

9. Compound, salt or solvate for use according to any preceding claim, wherein R₃ is H or a phenyl ring, or a 6- or 5-membered heteroaromatic ring containing one or more N heteroatoms; and wherein the ring is unsubstituted or substituted at one or more available positions with NH₂, OH, halogen or C₁-C₆ alkyl.

10. Compound, salt or solvate for use according to any preceding claim, wherein R₃ is H, or phenyl or pyridyl; wherein the rings are unsubstituted or substituted at one or more available positions with NH₂, halogen or C₁-C₆ alkyl.

11. Compound for use according to claim 1, wherein the compound is: 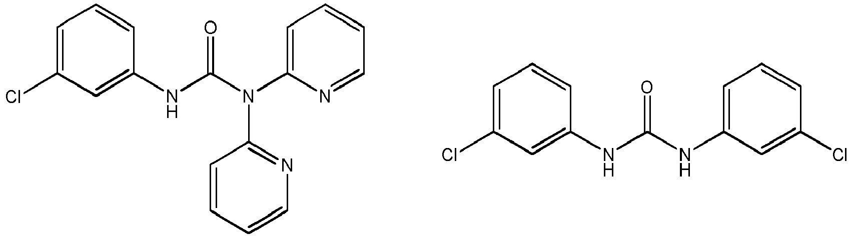 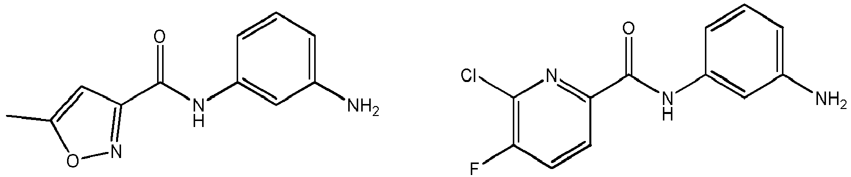 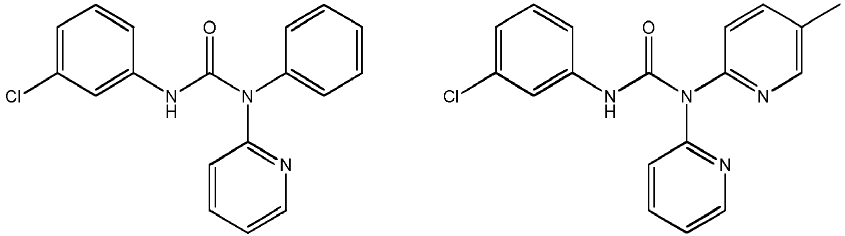 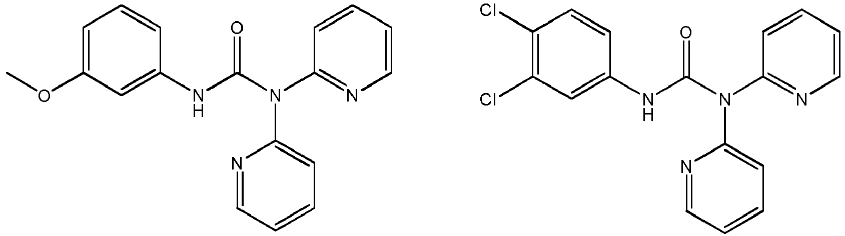 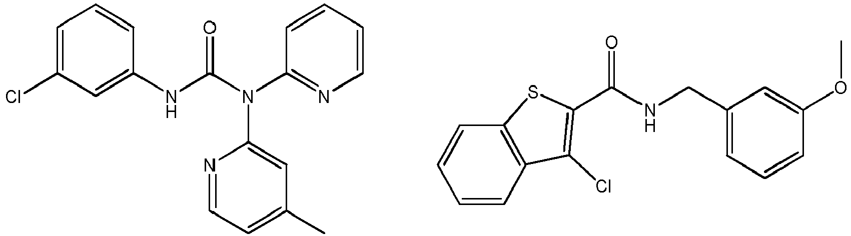 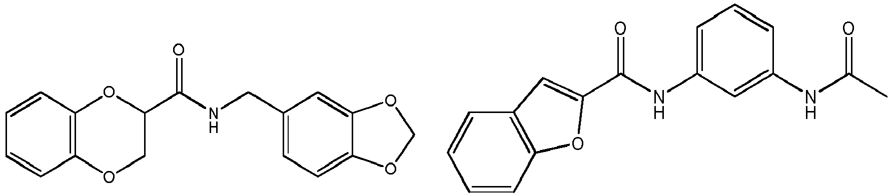 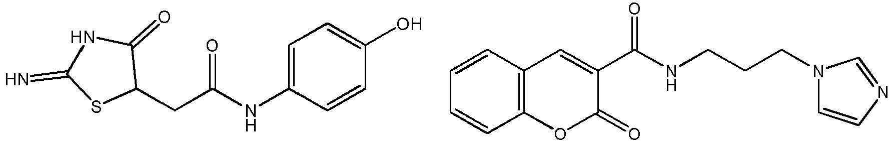 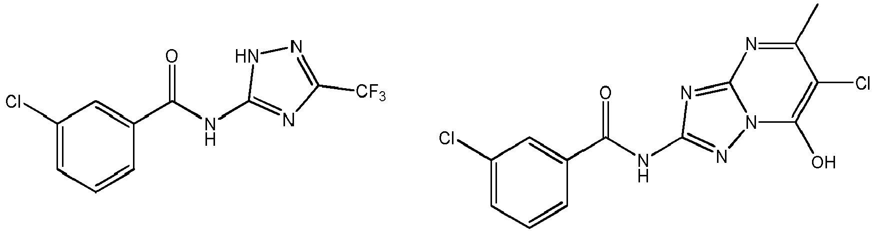 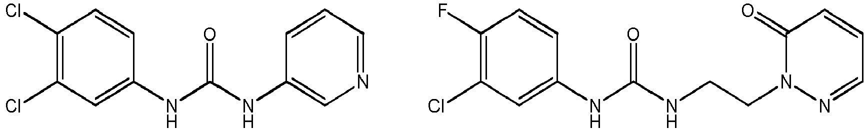 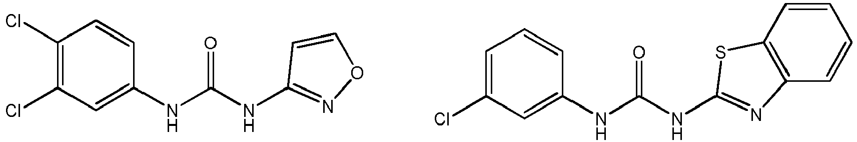 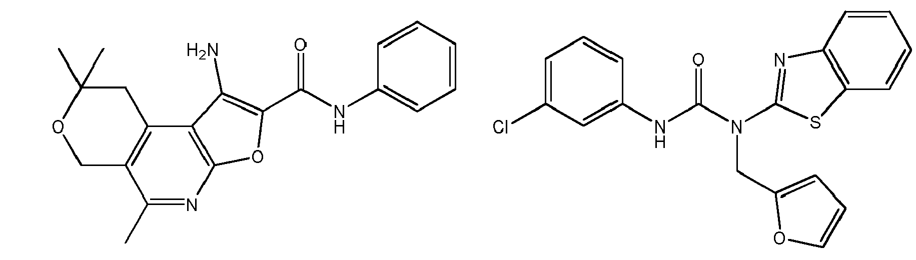 or 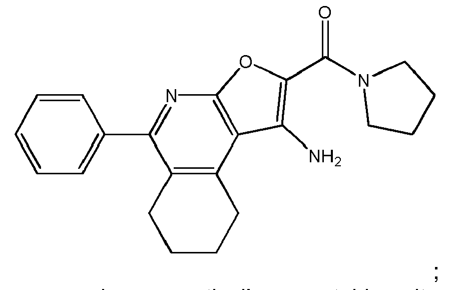 or a pharmaceutically acceptable salt or solvate thereof.

12. Compound for use according to claim 1, wherein the compound is: 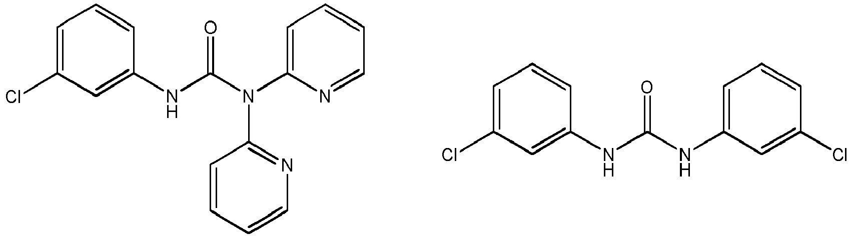 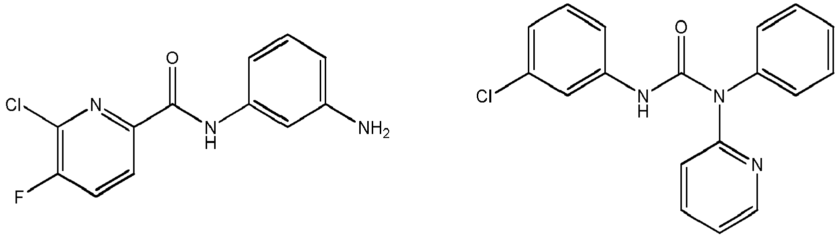 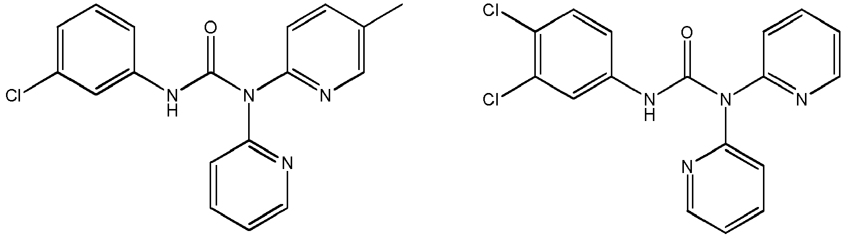 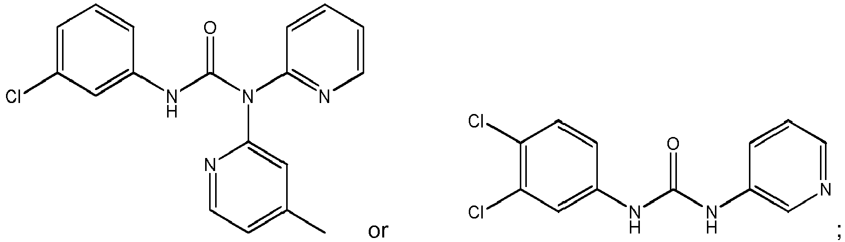 or a pharmaceutically acceptable salt or solvate thereof.

13. Compound for use according to claim 1, wherein the compound is: 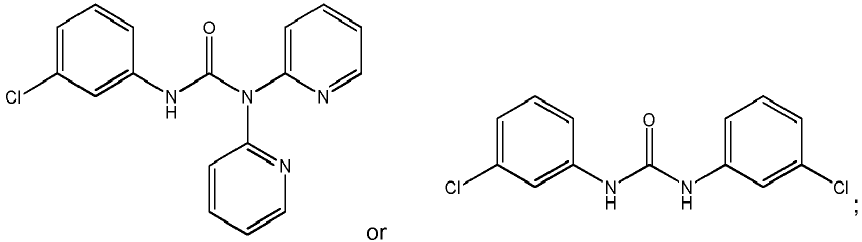 or a pharmaceutically acceptable salt or solvate thereof.

14. Compound, salt or solvate for use according to any preceding claim, for use in the treatment of a congenital disorder of glycosylation, preferably PMM2-CDG.

15. Pharmaceutical composition comprising a compound of formula (I) or a pharmaceutically acceptable salt or solvate thereof as defined in any of claims 1-13, and a pharmaceutically acceptable excipient.
